(19) Europäisches Patentamt
European Patent Office
Office européen des brevets

(11) Veröffentlichungsnummer: **0 082 369**
**B1**

(12) # EUROPÄISCHE PATENTSCHRIFT

(45) Veröffentlichungstag der Patentschrift: **09.04.86**

(51) Int. Cl.⁴: **C 07 D 471/04**, A 61 K 31/44 // (C07D471/04, 235:00, 221:00)

(21) Anmeldenummer: **82111185.3**

(22) Anmeldetag: **03.12.82**

(54) **Imidazo(4,5-c)pyridine, diese enthaltende pharmazeutische Zubereitungen und Verfahren zu ihrer Herstellung.**

(30) Priorität: **19.12.81 DE 3150486**

(43) Veröffentlichungstag der Anmeldung: **29.06.83 Patentblatt 83/26**

(45) Bekanntmachung des Hinweises auf die Patenterteilung: **09.04.86 Patentblatt 86/15**

(84) Benannte Vertragsstaaten: **AT BE CH DE FR GB IT LI NL SE**

(56) Entgegenhaltungen:
**US - A - 3 891 660**

**RECUEIL DES TRAVAUX CHIMIQUES DES PAYS-BAS, Band 90, Nr. 11, 1971, Den Haag, NL. K.B. DE ROOS et al.: "Deazapurine derivatives. VII. Synthesis of substituted imidazo- and triazolo-pyridines"**

(73) Patentinhaber: **Merck Patent Gesellschaft mit beschränkter Haftung, Frankfurter Strasse 250, D-6100 Darmstadt (DE)**

(72) Erfinder: **Irmscher, Klaus, Dr., Reuterallee 10, D-6100 Darmstadt (DE)**
Erfinder: **Saiko, Otto, Dr., Mozartweg 14, D-6100 Darmstadt (DE)**
Erfinder: **Minck, Klaus-Otto, Dr., Büchestrasse 8, D-6105 Ober-Ramstadt (DE)**
Erfinder: **Wolf, Hans-Peter, Prof. Dr., Brückenweg 6, D-6146 Alsbach (DE)**

**EP 0 082 369 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Imidazo(4,5-c)pyridine der allgemeinen Formel I

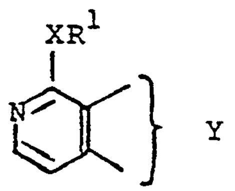

I

worin

X O, S oder NH,

Y $-NR^2-CH=N-$ oder $-N=CH-NR^2-$,

$R^1$ 1-Benzothienyl-methyl, Naphthylmethyl oder ein- oder zweifach durch F, Cl, $NO_2$ und/oder $CF_3$ substituiertes Benzyl und

$R^2$ Alkyl mit 1 - 4 C-Atomen oder Cyclopropylmethyl

bedeuten,

sowie deren physiologisch unbedenkliche Säureadditionssalze.

Die Formel I umschließt die 1H-Imidazo(4,5-c)-pyridine Formel Ia; = Formel I, Y = $-N=CH-NR^2-$; vgl. "The Ringndex", 2nd Edition, American Chemical Society, 1960, r. 1194) und die 3H-Imidazo(4,5-c)pyridine (Formel Ib; Formel I, Y = $-NR^2-CH=N-$; vgl. "The Ring-Index", 1.c., Nr. 1195).

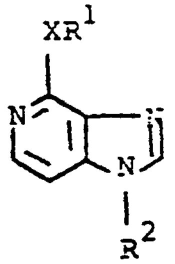

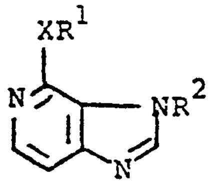

Ia Ib

Von dieser sind die Verbindungen der Formel Ib bevorzugt

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I und ihrer physiologisch unbedenklichen Salze gelöst.

Es wurde gefunden, daß diese Verbindungen bei guter Verträglichkeit wertvolle pharmakologische Eigenschaften besitzen. Insbesondere treten selektiv GABA-agonistische (gemessen an der Hemmung bicucullininduzierter Krämpfe, z.B. nach der Methode von D.R. Curtis et al., Brain Res., Band 33 (1971), Seiten 57 ff. und M. perez de la Mora, Biochem. pharmacology, Band 22 (1973) Seiten 2635 ff.) und/oder benzodiazepin-antagonistische Wirkungen auf;die Substanzen zeigen in vitro eine starke Bindung an Benzodiazepin-Rezeptoren (feststellbar z.B. nach der Methode von R.F. Squires und C. Braestrup, Nature (London), Band 266 (1977), Seiten 732-734 sowie H. Möhler und T. Okada, Life Sciences, Band 20 (1977), Seiten 2101-2110; Science (Washington, D.C.), Band 198 (1977), Seiten 849-851). Weiterhin zeigen sich anorektische Wirkungen (nach der Methode von Levine und Morley, Science (Washington, D.C.), Band 217 (1982) Seite 77).

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Gegenstand der Erfindung sind Verbindungen der Formel I und ihre physiologisch unbedenklichen Salze.

In den Verbindungen der Formel I steht der Rest X vorzugsweise für O oder S.

Der Rest $R^1$ bedeutet bevorzugt 1-Benzothienyl-3-methyl, o-, m- oder p-Fluorbenzyl, o-, m- oder p-Chlorbenzyl, o-, m- oder p-Nitrobenzyl, o-, m- oder p-Trifluormethylbenzyl, 2,3-, 2,4-, 2,5-, 2,6-, 3,4- oder 3,5-Dichlor-benzyl, ferner 1-Benzothienyl-2-methyl, Naphthyl-1- oder -2-methyl, 2,3-, 2,4-, 2,5-, 2,6-, 3,4- oder 3,5-Difluorbenzyl, 2,3-, 2,4-, 2,5-, 2,6-, 3,4- oder 3,5-Dinitrobenzyl, 2,3-, 2,4-, 2,5-, 2,6-, 3,4- oder 3,5-Bis-(trifluormethyl)-benzyl, 2-Chlor-3-, -4-, -5- oder -6-fluor-benzyl, 3-Chlor-2-, -4-, -5- oder -6-fluorbenzyl, 4-Chlor-2- oder -3-fluorbenzyl, 2-Fluor-3-, -4-, -5- oder -6-nitro-benzyl, 3-Fluor-2-,-4-, -5- oder -6-nitrobenzyl, 4-Fluor-2- oder -3-nitrobenzyl, 2-Fluor-3-, -4-, -5- oder -6-trifluormethylbenzyl, 3-Fluor-2-, -4-, -5- oder -6-trifluormethylbenzyl, 4-Fluor-2- oder -3-trifluormethylbenzyl, 2-Chlor-3-, -4-, -5- oder -6-nitrobenzyl, 3-Chlor-2-, -4-, -5- oder -6-nitrobenzyl, 4-Chlor-2- oder -3-nitro-benzyl, 2-Chlor-3-, -4-, -5- oder -6-trifluormethylbenzyl, 3-Chlor-2-, -4-, -5- oder -6-trifluormethylbenzyl, 4-Chlor-2- oder -3-trifluormethylbenzyl, 2-Nitro-3-, -4-, -5-oder -6-trifluormethylbenzyl, 3-Nitro-2-, -4-, -5- oder -6-trifluormethylbenzyl, 4-Nitro-2- oder -3-trifluor-methylbenzyl.

Der Rest $R^2$ bedeutet vorzugsweise Methyl, ferner Ethyl oder Cyclopropylmethyl, weiterhin propyl, Isopropyl, Butyl, Isobutyl, sek:Butyl oder tert.-Butyl.

Gegenstand der Erfindung sind insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ic bis Ie ausgedrückt werden, die der Formel I bzw. Ia oder Ib entsprechen, worin jedoch

in Ic X O bedeutet;
in Id X S bedeutet;
in Ie X NH bedeutet.

Weitere bevorzugte Gruppen von Verbindungen sind die der Teilformeln If bis Ih, die den Formeln I bzw. Ia bis Ie entsprechen, worin jedoch
in If $R^1$ 1-Benzothienylmethyl, Fluorbenzyl, Chlorbenzyl, Trifluormethylbenzyl, Dichlorbenzyl oder Chlorfluorbenzyl bedeutet;
in Ig $R^1$ 1-Benzothienyl-3-methyl, o-Fluorbenzyl, o-Chlorbenzyl, o-Trifluormethylbenzyl, 2,4- oder 2,6-Dichlorbenzyl oder 2-Chlor-6-fluorbenzyl bedeutet;
in Ih $R^1$ 1-Benzothienyl-3-methyl bedeutet;
in Ii $R^1$ 1-Benzothienyl-3-methyl und $R^2$ Methyl bedeutet.

Gegenstand der Erfindung ist ferner ein Verfahren zur Herstellung der Verbindungen der Formel I sowie ihrer physiologisch unbedenklichen Salze, dadurch gekennzeichnet, daß man eine Verbindung der allgemeinen Formel II

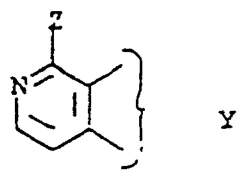 II

worin
Z F, Cl, Br, J, OH, SH, $SR^3$, $SOR^3$, $SO_2R^3$ oder $NH_2$ und
$R^3$ Alkyl mit 1 - 4 C-Atomen, phenyl oder Benzyl bedeuten und
Y die angegebene Bedeutung hat oder eines ihrer reaktionsfähigen Derivate mit einer Verbindung der allgemeinen Formel III

$HX-R^1$ III

worin
X und $R^1$ die angegebene Bedeutung haben oder einem ihrer reaktionsfähigen Derivate umsetzt
oder daß man eine Verbindung der allgemeinenen Formel IV

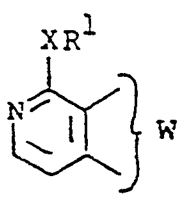 IV

worin
W -NH-CH=N- oder -N=CE-NH- bedeutet mit einer Verbindung der allgemeinen Formel V

$Z-R_2$ V

worin
$R^2$ und Z die ancegebene Bedeutung haben oder einem ihrer reaktionsfähigen Derivate umsetzt
oder daß man eine Verbindung der allgemeinen Formel VI

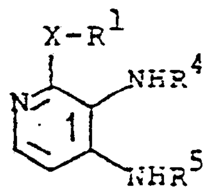 VI

worin
der eine der Reste $R^4$ bzw. $R^5$ H,
der andere dieser Reste $R^2$ bedeutet und
X und $R^1$ die angegebene Bedeutung haben mit Ameisensäure oder einem ihrer reaktionsfähigen Derivate umsetzt
und/oder daß man gegebenenfalls eine Verbindung der allgemeinen Formel I durch Behandeln mit einer Säure in eines ihrer physiologisch unbedenklichen Säureadditionssalze umwandelt.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Vorzugsweise werden die Verbindungen der Formel I erhalten durch Reaktion von Imidazo(4,5-c)pyridinen der Formel II oder ihrer reaktionsfähigen Derivate (z.B. den entsprechenden Metallalkoholaten oder Metallmercaptiden) mit verbindungen der Formel III oder ihren reaktionsfähigen Derivaten (z.B. den entsprechenden Metallalkoholaten, Metallmercaptiden, reaktionsfähigen Estern, insbesondere den entsprechenden Chloriden oder Bromiden der Formeln $R^1Cl$ oder $R^1Br$).

Die Ausgangsstoffe der Formeln II und III sind teilweise bekannt. Sofern sie nicht bekannt sind, können sie nach an sich bekannten Methoden hergestellt werden. 8o sind die 1H-Imidazo(4,5-c)pyridine der Formel II beispielsweise durch Kondensation von 2-Z-3-amino-4-$NHR^2$-pyridinen, die 3H-Imidazo(4,5-c)pyridine der Formel II durch

Kondensation von 2-Z-3-NHR$^2$-4-aminopyridinen mit Ameisensäure/ Acetanhydrid erhältlich. In den Verbindungen der Formel II bedeutet der Rest Z vorzugsweise ein Chloratom.

Im einzelnen erfolgt die Umsetzung von II mit III in Gegenwart oder Abwesenheit eines inerten Lösungsmittels bei Temperaturen zwischen etwa −20 und 250°, vorzugsweise zwischen 60 und 150°. Als Lösungsmittel eignen sich beispielsweise Kohlenwasserstoffe wie Benzol, Toluol, Xylole oder Mesitylen; tertiäre Basen wie Triethylamin; pyridin oder picoline; Alkohole wie Methanol, Ethanol oder Butanol; Glykole und Glykolether wie Ethylenglykol, Diethylenglykol, 2-Methoxyethanol; Ketone wie Aceton; Ether wie Tetrahydrofuran oder Dioxan; Amide wie Dimethylformamid; Sulfoxide wie Dimethylsulfoxid. Auch Gemische dieser Lösungsmittel sind geeignet. Alkohole und Mercaptane der Formel III (X = O oder S) werden zweckmäßig zunächst in die entsprechenden Alkoholate bzw. Mercaptide umgewandelt,vorzugsweise durch Reaktion mit Natrium, Kalium, Natrium- oder Kaliumethylat, Natrium- oder Kaliumhydrid in die entsprechenden Natriumoder Kaliumalkoholate bzw. -mercaptide.

Für eine Umsetzung ohne Lösungsmittel in der Schmelze eignen sich insbesondere die Amine der Formel III (X = NH). Ihre Umsetzung mit Verbindungen der Formel II (Z = Cl) gelingt beispielsweise durch mehrstündiges Erhitzen auf etwa 160 - 190° mit Vorteil verwendet man einen Überschuß des Amins.

Die Verbindungen der Formel I sind ferner erhältlich durch N-Alkylierung der Imidazo(4,5-c)pyridine der Formel IV mit Verbindungen der Formel V oder ihren reaktionsfähigen Derivaten. Die Ausgangsstoffe der Formel IV sind neu, aber in an sich bekannter Weise erhältlich, beispielsweise durch Umsetzung von 2-Z-3,4-diamino-pyridinen mit Ameisensäure/Acetanhydrid zu 2-Z-imidazo(4,5-c)-pyridinen und anschließende Reaktion mit Verbindungen der Formel III nach den oben angegebenen Methoden; die beiden Reaktionsschritte können auch vertauscht werden, die Synthese durchläuft dann die entsprechenden 2-XR$^1$-3,4-diaminopyridine. Als Verbindungen der Formel V eignen sich in erster Linie die entsprechenden Chloride, Bromide und Jodide, z.B. Methylchlorid, -bromid oder -jodid, Ethylchlorid, -bromid oder -jodid, Cyclopropylmethylchlorid, -bromid oder -jodid, ferner andere reaktive Ester von Alkoholen der Formel R$^2$-OH, z.B. Methyl-p-toluolsulfonat oder Dimethylsulfat. Die N-Alkylierung gelingt z.B. in Gegenwart eines der oben angegebenen Lösungsmittel (z.B. Ethanol oder Aceton) bei Temperaturen zwischen etwa 0 und 100°. Zweckmäßig ist der Zusatz einer Base wie Kaliumcarbonat.

Weiterhin sind die Verbindungen der Formel I erhältlich durch Umsetzung von Diaminopyridinen der Formel VI (herstellbar z.B. durch Reaktion eines 2-Z-3-NHR$^4$-4-NHR$^5$-pyridins mit einer Verbindung der Formel III unter den oben angegebenen Bedingungen) mit Ameisensäure oder einem ihrer reaktionsfähigen Derivate, z.B. s-Triazin oder einem Orthoameisensäuretrialkylester, worin die Alkylgruppe vorzugsweise 1-4 C-Atome besitzt. Die Umsetzung verläuft zweckmäßig in Gegenwart oder Abwesenheit eines der genannten inerten Lösungsmittel bei Temperaturen zwischen etwa 20 und etwa 200°. Der Zusatz eines Kondensationsmittels wie Acetanhydrid kann vorteilhaft sein. Mit s-Triazin arbeitet man zweckmäßig ohne Lösungsmittel, beispielsweise unter den in der US-PS 28 41 585 angegebenen Bedingungen.

Eine Base der Formel I kann mit einer Säure in das zugehörige Säureadditionssalz überführt werden. Für diese Umsetzung kommen Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, phosphorsäuren wie Orthophosphorsäure, Sulfaminsäure, ferner organische Säuren, insbesondere aliphatische, alicyclische, araliphatische, aromatische oder heterocyclische einoder mehrbasige Carbon-, 8ulfon- oder Schwefelsäuren, z. B. Ameisensäure, Essigsäure, propionsäure, pivalin-Säure, Diethylessigsäure, Malonsäure, Bernsteinsäure, pimelinsäure, Fumarsäure, Maleinsäure, Milchsäure, Weinsäure, Äpfelsäure, Benzoesäure, Salicylsäure, 2-oder 3-phenylpropionsäure, Citronensäure, Gluconsäure, Ascorbinsäure, Nicotinsäure, Isonicotinsäure, Methanoder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise wasser, pflanzliche Ole, Benzylalkohole,

polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I können bei der Bekämpfung von Krankheiten verwendet werden, insbesondere von Krankheitszuständen, die mit Hilfe von GABA-Agonisten beeinflußt werden können, z.B. Hirninfarkt, Epilepsie, Spätdyskinesie, Huntington's Chorea, Abstinenzsyndrom bei Alkoholikern, Alkoholentzugskrämpfe, spastische Zustände, Angst, Schlafstörungen, Schmerz, allgemein bei der prophylaxe des krampfbedingten bzw. alkoholbedingten Zelluntergangs, weiterhin zur Beeinflussung der physiologischen Müdigkeit, ferner zur Antagonisierung der zentral dämpfenden, muskelrelaxierenden, ataktischen, blutdrucksenkenden und atemdepressiven Eigenschaften von tranquillierend wirksamen Benzodiazepinen, insbesondere 1,4- und 1,5-Benzodiazepinen, sowie zur Antagonisierung von Nicht-Benzodiazepinen, soweit sie agonistisch an dem Benzodiazepin-Rezeptor angreifen, insbesondere zur Antagonisierung der sedierenden Wirkung anderer Arzneimittel, beispielsweise als Antidot bei Intoxikationen, bei welchen übermäßige Einnahme von Benzodiazepinen beteiligt ist, oder zur Abkürzung einer durch Benzodiazepine eingeleiteten Anästhesie, sowie ihre Verwendung bei der therapeutischen Behandlung des menschlischen oder tierischen Körpers.

Dabei werden die erfindungsgemäßen Substanzen in der Regel in Analogie zu bekannten wirksamen Substanzen, wie phenytoin oder Progabid, verabreicht, vorzugsweise in Dosierungen zwischen etwa 1 und 500 mg, insbesondere zwischen 5 und 100 mg pro Dosierungseinheit. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindungen, vom Alter, Körpergewicht, allgemeinen Gesundheitszustnad, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatographie und/oder Kristallisation.

Vor- und nachstehend sind alle Temperaturen in Celsiusgraden angegeben.

**Beispiel 1**

Man löst 15,84 g 1-Benzothienyl-3-methanol in 110 ml DMF, gibt 2,88 g NaH hinzu und rührt 1 Stunde bei 20°. Nach Zugabe einer Lösung von 16,76 g 4-Chlor-1-methyl-1H-imidazo(4,5-c)pyridin [F. 132-134; erhältlich durch Umsetzung von 4-Methylamino-3-nitropyridin mit $HCl/SnCl_2$ zu 3-Amino-2-chlor-4-methylaminopyridin (F. 170 173°) und Reaktion mit HCOOH/Acetanhydrid] in 70 ml DMF wird 15 Stunden bei 90 - 95° gerührt. Man dampft ein, arbeitet wie üblich auf und erhält 4-(1-Benzothienyl-3-meth-oxy)-1-methyl-1H-imidazo(4,5-c)pyridin, F. 200 - 204° (aus Ethylacetat/Tetrahydrofuran).

**Beispiel 2**

Analog Beispiel 1 erhält man aus 1-Benzothienyl-3-methanol und 4-Chlor-3-methyl-3H-imidazo(4,5-c)pyridin [F. 160-161°; erhältlich durch Umsetzung von 4-Amino-2-chlor-3-p-toluolsulfonamidopyridin mit Dimethylsulfat/$K_2CO_3$ in Aceton zu 4-Amino-2-chlor-3-methylamino-pyridin (F. 62 - 65°) und Reaktion mit HCOOH/Acetanhydrid] das 4-(1-Benzothienyl-3-methoxy)-3-methyl-3H-imidazo(4,5-c)-pyridin, F. 160 - 162°

**Beispiele 3 bis 62**

Analog Beispiel 1 erhält man aus den entsprechenden 4-Chlor-imidazo(4,5-c)pyridinen mit den entsprechenden Alkoholen:

3. 4-(1-Benzothienyl-2-methoxy)-1-methyl-1H-imidazo-(4,5-c)pyridin, F. 156-158°.

4. 4-(Naphthyl-1-methoxy)-1-methyl-1H-imidazo(4,5-c)-pyridin, F. 199-200°.

5. 4-(Naphthyl-2-methoxy)-1-methyl-1H-imidazo(4,5-c)-pyridin, F. 109-110°.

6. 4-o-Fluorbenzyloxy-1-methyl-1H-imidazo(4,5-c)pyridin, F. 108 110°.
7. 4-m-Fluorbenzyloxy-1-methyl-1H-imidazo(4,5-c)pyridin.
8. 4-p-Fluorbenzyloxy-1-methyl-1H-imidazo(4,5-c)pyridin.
9. 4-o-Chlorbenzyloxy-1-methyl-1H-imidazo(4,5-c)pyridin, F. 132-135°.
10. 4-m-Chlorbenzyloxy-1-methyl-1H-imidazo(4,5-c)pyridin.
11. 4-p-Chlorbenzyloxy-1-methyl-1H-imidazo(4,5-c)pyridin, F. 142-146°.
12. 1-Methyl-4-o-nitrobenzyloxy-1H-imidazo(4,5-c)-pyridin.
13. 1-Methyl-4-m-nitrobenzyloxy-1H-imidazo(4,5-c)-pyridin.
14. 1-Methyl-4-p-nitrobenzyloxy-1H-imidazo(4,5-c)-pyridin.
15. 1-Methyl-4-o-trifluormethylbenzyloxy-1H-imidazo(4,5-c)pyridin, F. 108 110°
16. 1-Methyl-4-m-trifluormethylbenzyloxy-1H-imidazo(4,5-c)pyridin.
17. 1-Methyl-4-p-trifluormethylbenzyloxy-1H-imidazo(4,5-c)pyridin.
18. 4-(2,3-Dichlorbenzyloxy)-1-methyl-1H-imidazo(4,5-c)-pyridin.
19. 4-(2,4-Dichlorbenzyloxy)-1-methyl-1H-imidazo(4,5-c)-pyridin. F. 130-132°.
20. 4-(2,5-Dichlorbenzyloxy)-1-methyl-1H-imidazo-(4,5-c)pyridin.
21. 4-(2,6-Dichlorbenzyloxy)-1-methyl-1H-imidazo-(4,5-c)pyridin.
22. 4-(3,4-Dichlorbenzyloxy)-1-methyl-1H-imidazo-(4,5-c)pyridin.
23. 4-(3,5-Dichlorbenzyloxy)-1-methyl-1H-imidazo-(4,5-c)pyridin.
24. 4-(2,4-Dinitrobenzyloxy)-1-methyl-1H-imidazo-(4,5-c)pyridin.
25. 4-(3,4-Dinitrobenzyloxy)-1-methyl-1H-imidazo-(4,5-c)pyridin.
26. 4-(3,5-Dinitrobenzyloxy)-1-methyl-1H-imidazo-(4,5-c)pyridin.
27. 4-(2-Chlor-6-fluorbenzyloxy)-1-methyl-1H-imidazo-(4,5-c)pyridin.
28. 4-(2-Chlor-4-nitrobenzyloxy)-1-methyl-1H-imidazo-(4,5-c)pyridin.
29. 4-(2-Chlor-5-nitrobenzyloxy)-1-methyl-1H-imidazo-(4,5-c)pyridin.
30. 4-(4-Chlor-2-nitrobenzyloxy)-1-methyl-1H-imidazo-(4,5-c)pyridin.
31. 4-(4-Chlor-3-nitrobenzyloxy)-1-methyl-1H-imidazo-(4,5-c)pyridin.
32. 4-(5-Chlor-2-nitrobenzyloxy)-1-methyl-1H-imidazo-(4,5-c)pyridin.
33. 4-(1-Benzothienyl-2-methoxy)-3-methyl-3H-imidazo-(4,5-c)pyridin.
34. 4-(Naphthyl-1-methoxy)-3-methyl-3H-imidazo(4,5-c)-pyridin, F.141-142°.
35. 4-(Naphthyl-2-methoxy)-3-methyl-3H-imidazo(4,5-c)-pyridin, F.171°.
36. 4-o-Fluorbenzyloxy-3:methyl-3H-imidazo(4,5-c)pyri-din, F. 142 144°
37. 4-m-Fluorbenzyloxy-3-methyl-3H-imidazo(4,5-c)pyri-din.
38. 4-p-Fluorbenzyloxy-3-methyl-3H-imidazo(4,5-c)pyri-din.
39. 4-o-Chlorbenzyloxy-3-methyl-3H-imidazo(4,5-c)pyri-din, F. 125-127°.
40. 4-m-Chlorbenzyloxy-3-methyl-3H-imidazo(4,5-c)pyri-din.
41. 4-p-Chlorbenzyloxy-3-methyl-3H-imidazo(4,5-c)pyri-din, F. 145-147°.
42. 3-Methyl-4-o-nitrobenzyloxy-3H-imidazo(4,5-c)pyri-din.
43. 3-Methyl-4-m-nitrobenzyloxy-3H-imidazo(4,5-c)pyri-din.
44. 3-Methyl-4-p-nitrobenzyloxy-3H-imidazo(4,5-c)pyri-din.
45. 3-Methyl-4-o-trifluormethylbenzyloxy-3H-imidazo-(4,5-c)pyridin.
46. 3-Methyl-4-m-trifluormethylbenzyloxy-3H-imidazo-(4,5-c)pyridin.
47. 3-Methyl-4-p-trifluormethylbenzyloxy-3H-imidazo-(4,5-c)pyridin.
48. 4-(2,3-Dichlorbenzyloxy)-3-methyl-3H-imidazo(4,5-c)-pyridin.
49. 4-(2,4-Dichlorbenzyloxy)-3-methyl-3H-imidazo(4,5-c)-pyridin,,F 139 140°
50. 4-(2,5-Dichlorbenzyloxy)-3-methyl-3H-imidazo(4,5-c)-pyridin.
51. 4-(2,6-Dichlorbenzyloxy)-3-methyl-3H-imidazo(4,5-c)-pyridin.
52. 4-(3,4-Dichlorbenzyloxy)-3-methyl-3H-imidazo(4,5-c)-pyridin.
53. 4-(3,5-Dichlorbenzyloxy)-3-methyl-3H-imidazo(4,5-c)-pyridin.
54. 4-(2,4-Dinitrobenzyloxy)-3-methyl-3H-imidazo(4,5-c)-pyridin.
55. 4-(3,4-Dinitrobenzyloxy)-3-methyl-3H-imidazo(4,5-c)-pyridin.
56. 4-(3,5-Dinitrobenzyloxy)-3-methyl-3H-imidazo(4,5-c)-pyridin.
57. 4-(2-Chlor-6-fluorbenzyloxy)-3-methyl-3H-imidazo-(4,5-c)pyridin.
58. 4-(2-Chlor-4-nitrobenzyloxy)-3-methyl-3H-imidazo-(4,5-c)pyridin.
59. 4-(2-Chlor-5-nitrobenzyloxy)-3-methyl-3H-imidazo-(4,5-c)pyridin.
60. 4-(4-Chlor-2-nitrobenzyloxy)-3-methyl-3H-imidazo-(4,5-c)pyridin.
61. 4-(4-Chlor-3-nitrobenzyloxy)-3-methyl-3H-imidazo-(4,5-c)pyridin.
62. 4-(5-Chlor-2-nitrobenzyloxy)-3-methyl-3H-imidazo-(4,5-c)pyridin.

**Beispiel 63**

Man löst 2,78 g Na in 180 ml Ethanol, gibt 19,1 g o-Chlorbenzylmercaptan und 16,78 g 4-Chlor-1-methyl-1H-imidazo(4,5-c)pyridin hinzu, kocht 16 Stunden, dampft ein, arbeitet wie üblich auf und erhält 4-o-Chlorbenzyl-thio-1-methyl-1H-imidazo(4,5-c)pyridin, F. 152 155° (aus Ethanol).

**Beispiele 64 bis 126**

Analog Beispiel 63 erhält man aus den entsprechenden 4-Chlor-imidazo(4,5-c)pyridinen mit den entsprechenden Mercaptanen:

64. 4-(1-Benzothienyl-3-methylthio)-1-methyl-1H-imidazo-(4,5-c)pyridin. F. 146-148°.
65. 4-(1-Benzothienyl-3-methylthio)-1-butyl-1H-imidazo-(4,5-c)pyridin, F. 157-159°.
66. 4-(1-Benzothienyl-3-methylthio)-1-cyclopropyl-methyl-1H-imidazo(4,5-c)pyridin. F. 103-104°.
67. 4-(1-Benzothienyl-2-methylthio)-1-methyl-1H-imidazo-(4,5-c)pyridin, F. 152-154°.
68. 4-(Naphthyl-1-methylthio)-1-methyl-1H-imidazo(4 5-c)-pyridin. F. 143-144°
69. 4-(Naphthyl-2-methylthio)-1-methyl-1H-imidazo(4,5-c)-pyridin. F. 156-158°.
70. 4-o-Fluorbenzylthio-1-methyl-1H-imidazo(4,5-c)pyridin.
71. 4-m-Fluorbenzylthio-1-methyl-1H-imidazo(4,5-c)pyridin.
72. 4-p-Fluorbenzylthio-1-methyl-1H-imidazo(4,5-c)pyridin.
73. 4-m-Chlorbenzylthio-1-methyl-1H-imidazo(4,5-c)pyridin.
74. 4-p-Chlorbenzylthio-1-methyl-1H-imidazo(4,5-c)pyridin. F. 151-153°.
75. 1-Methyl-4-o-nitrobenzylthio-1H-imidazo(4,5-c)pyridin.
76. 1-Methyl-4-m-nitrobenzylthio-1H-imidazo(4,5-c)pyridin.
77. 1-Methyl-4-p-nitrobenzylthio-1H-imidazo(4,5-c)pyridin.
78. 1-Methyl-4-o-trifluormethylbenzylthio-1H-imidazo-(4,5-c)pyridin.
79. 1-Methyl-4-m-trifluormethylbenzylthio-1H-imidazo-(4,5-c)pyridin.
80. 1-Methyl-4-p-trifluormethylbenzylthio-1H-imidazo-(4,5-c)pyridin.
81. 4-(2,3-Dichlorbenzylthio)-1-methyl-1H-imidazo(4,5-c)-pyridin.
82. 4-(2,4-Dichlorbenzylthio)-1-methyl-1H-imidazo(4,5-c)-pyridin. F. 126-128°.
83. 4-(2,5-Dichlorbenzylthio)-1-methyl-1H-imidazo(4,5-c)-pyridin.
84. 4-(2,6-Dichlorbenzylthio)-1-methyl-1H-imidazo(4,5-c)-pyridin.
85. 4-(3,4-Dichlorbenzylthio)-1-methyl-1H-imidazo(4,5-c)-pyridin.
86. 4-(3,5-Dichlorbenzylthio)-1-methyl-1H-imidazo(4,5-c)-pyridin.
87. 4-(2,4-Dinitrobenzylthio)-1-methyl-1H-imidazo-(4,5-c)pyridin.
88. 4-(3,4-Dinitrobenzylthio)-1-methyl-1H-imidazo-(4,5-c)pyridin.
89. 4-(3,5-Dinitrobenzylthio)-1-methyl-1H-imidazo-(4,5-c)pyridin.
90. 4-(2-Chlor-6-fluorbenzylthio)-1-methyl-1H-imidazo-(4,5-c)pyridin.
91. 4-(2-Chlor-4-nitrobenzylthio)-1-methyl-1H-imidazo-(4,5-c)pyridin.
92. 4-(2-Chlor-5-nitrobenzylthio)-1-methyl-1H-imidazo-(4,5-c)pyridin.
93. 4-(4-Chlor-2-nitrobenzylthio)-1-methyl-1H-imidazo-(4,5-c)pyridin.
94. 4-(4-Chlor-3-nitrobenzylthio)-1-methyl-1H-imidazo-(4,5-c)pyridin.
95. 4-(5-Chlor-2-nitrobenzylthio)-1-methyl-1H-imidazo-(4,5-c)pyridin.
96. 4-(1-Benzothienyl-3-methylthio)-3-methyl-3H-imidazo-(4,5-c)pyridin, F. 144-147°.Hemimalonat, F.139 140° Hemimaleat, F.135 137°
97. 4-(1-Benzothienyl-2-methylthio)-3-methyl-3H-imidazo-(4,5-c)pyridin.
98. 4-(Naphthyl-1-methylthio)-3-methyl-3H-imidazo(4,5-c)-pyridin, F. 159-160°.
99. 4-(Naphthyl-2-methylthio)-3-methyl-3H-imidazo(4,5-c)-pyridin, F. 119-120°.
100. 4-o-Fluorbenzylthio-3-methyl-3H-imidazo(4,5-c)-pyridin, F. 93 94°
101. 4-m-Fluorbenzylthio-3-methyl-3H-imidazo(4,5-c)-pyridin.
102. 4-p-Fluorbenzylthio-3-methyl-3H-imidazo(4,5-c)-pyridin.
103. 4-o-Chlorbenzylthio-3-methyl-3H-imidazo(4,5-c)-pyridin, F. 119-120°.
104. 4-m-Chlorbenzylthio-3-methyl-3H-imidazo(4,5-c)-pyridin.
105. 4-p-Chlorbenzylthio-3-methyl-3H-imidazo(4,5-c)-pyridin, F. 118-119°.
106. 3-Methyl-4-o-nitrobenzylthio-3H-imidazo(4,5-c)-pyridin.
107. 3-Methyl-4-m-nitrobenzylthio-3H-imidazo(4,5-c)-pyridin.
108. 3-Methyl-4-p-nitrobenzylthio-3H-imidazo(4,5-c)-pyridin.
109. 3-Methyl-4-o-trifluormethylbenzylthio-3H-imidazo-(4,5-c)pyridin, F. 128-128°.
110. 3-Methyl-4-m-trifluormethylbenzylthio-3H-imidazo-(4,5-c)pyridin.
111. 3-Methyl-4-p-trifluormethylbenzylthio-3H-imidazo-(4,5-c)pyridin.
112. 4-(2,3-Dichlorbenzylthio)-3-methyl-3H-imidazo-(4,5-c)pyridin.
113. 4-(2,4-Dichlorbenzylthio)-3-methyl-3H-imidazo-(4,5-c)pyridin, F. 101-102°.
114. 4-(2,5-Dichlorbenzylthio)-3-methyl-3H-imidazo-(4,5-c)pyridin.
115. 4-(2,6-Dichlorbenzylthio)-3-methyl-3H-imidazo-(4,5-c)pyridin, F. 148-150°.
116. 4-(3,4-Dichlorbenzylthio)-3-methyl-3H-imidazo-(4,5-c)pyridin.
117. 4-(3,5-Dichlorbenzylthio)-3-methyl-3H-imidazo-(4,5-c)pyridin.
118. 4-(2,4-Dinitrobenzylthio)-3-methyl-3H-imidazo-(4,5-c)pyridin.
119. 4-(3,4-Dinitrobenzylthio)-3-methyl-3H-imidazo-(4,5-c)pyridin.
120. 4-(3,5-Dinitrobenzylthio)-3-methyl-3H-imidazo-(4,5-c)pyridin.
121. 4-(2-Chlor-6-fluorbenzylthio)-3-methyl-3H-imidazo-(4,5-c)pyridin, F. 137 139°
122. 4-(2-Chlor-4-nitrobenzylthio)-3-methyl-3H-imidazo-(4,5-c)pyridin.
123. 4-(2-Chlor-5-nitrobenzylthio)-3-methyl-3H-imidazo-(4,5-c)pyridin.
124. 4-(4-Chlor-2-nitrobenzylthio)-3-methyl-3H-imidazo-(4,5-c)pyridin.
125. 4-(4-Chlor-3-nitrobenzylthio)-3-methyl-3H-imidazo-(4,5-c)pyridin.

126. 4-(5-Chlor-2-nitrobenzylthio)-3-methyl-3H-imidazo-(4,5-c)pyridin.

**Beispiel 127**

Eine Lösung von 16,76 g 4-Chlor-1-methyl-1H-imidazo(4,5-c)-pyridin und 35 g o-Chlorbenzylamin in 500 ml Xylol wird 24 Stunden gekocht. Beim Abkühlen kristallisiert 4-o-Chlorbenzylamino-1-methyl-1H-imidazo(4,5-c)pyridin aus; F. 173 176°

**Beispiel 128**

Ein Gemisch von 16,76 g 4-Chlor-3-methyl-3H-imidazo-(4,5-c)pyridin und 30 g o-Fluorbenzylamin wird 2 Stunden bei 180° geschmolzen. Nach üblicher Aufarbeitung erhält man 4-o-Fluorbenzylamino-3-methyl-3H-imidazo(4,5-c)pyridin, F. 163-164°.

**Beispiele 129 bis 188**

Analog Beispiel 127 erhält man aus den entsprechenden 4-Chlor-imidazo(4,5-c)pyridinen mit den entsprechenden Aminen:

129. 4-(1-Benzothienyl-3-methylamino)-1-methyl-1H-imidazo(4,5-c)pyridin,F.120-125°.Hydroch1orid,F.308-310°.

130. 4-(1-Benzothienyl-2-methylamino)-1-methyl-1H-imidazo(4,5-c)pyridin, F. 180-182°.

131. 4-(Naphthyl-1-methylamino)-1-methyl-1H-imidazo-(4,5-c)pyridin, F. 152-154°; Hydrochlorid, F.317-318°.

132. 4-(Naphthyl-2-methylamino)-1-methyl:1H-imidazo-(4,5-c)pyridin.

133. 4-o-Fluorbenzylamino-1-methyl-1H-imidazo(4,5-c)-pyridin.

134. 4-m-Fluorbenzylamino-1-methyl-1H-imidazo(4,5-c)-pyridin.

135. 4-p-Fluorbenzylamino-1-methyl-1H-imidazo(4,5-c)-pyridin.

136. 4-m-Chlorbenzylamino-1-methyl-1H-imidazo(4,5-c)-pyridin.

137. 4-p-Chlorbenzylamino-1-methyl-1H-imidazo(4,5-c)-pyridin.

138. 1-Methyl-4-o-nitrobenzylamino-1H-imidazo(4,5-c)-pyridin.

139. 1-Methyl-4-m-nitrobenzylamino-1H-imidazo(4,5-c)-pyridin.

140. 1-Methyl-4-p-nitrobenzylamino-1H-imidazo(4,5-c)-pyridin.

141. 1-Methyl-4-o-trifluormethylbenzylamino-1H-imidazo-(4,5-c)pyridin.

142. 1-Methyl-4-m-trifluormethylbenzylamino-1H-imidazo-(4,5-c)pyridin.

143. 1-Methyl-4-p-trifluormethylbenzylamino-1H-imidazo-(4,5-c)pyridin.

144. 4-(2,3-Dichlorbenzylamino)-1-methyl-1H-imidazo-(4,5-c)pyridin.

145. 4-(2,4-Dichlorbenzylamino)-1-methyl-1H-imidazo-(4,5-c)pyridin, F. 150-153°.

146. 4-(2,5-Dichlorbenzylamino)-1-methyl-1H-imidazo-(4,5-c)pyridin.

147. 4-(2,8-Dichlorbenzylamino)-1-methyl-1H-imidazo-(4,5-c)pyridin.

148. 4-(3,4-Dichlorbenzylamino)-1-methyl-1H-imidazo-(4,5-c)pyridin.

149. 4-(3,5-Dichlorbenzylamino)-1-methyl-1H-imidazo-(4,5-c)pyridin.

150. 4-(2,4-Dinitrobenzylamino)-1-methyl-1H-imidazo-(4,5-c)pyridin.

151. 4-(3,4-Dinitrobenzylamino)-1-methyl-1H-imidazo-(4,5-c)pyridin.

152. 4-(3,5-Dinitrobenzylamino)-1-methyl-1H-imidazo-(4,5-c)pyridin.

153. 4-(2-Chlor-6-fluorbenzylamino)-1-methyl-1H-imidazo-(4,5-c)pyridin.

154. 4-(2-Chlor-4-nitrobenzylamino)-1-methyl-1H-imidazo-(4,5-c)pyridin.

155. 4-(2-Chlor-5-nitrobenzylamino)-1-methyl-1H-imidazo-(4,5-c)pyridin.

156. 4-(4-Chlor-2-nitrobenzylamino)-1-methyl-1H-imidazo-(4,5-c)pyridin.

157. 4-(4-Chlor-3-nitrobenzylamino)-1-methyl-1H-imidazo-(4,5-c)pyridin.

158. 4-(5-Chlor-2-nitrobenzylamino)-1-methyl-1H-imidazo-(4,5-c)pyridin.

159. 4-(1-Benzothienyl-3-methylamino)-3-methyl-3H-imidazo(4,5-c)pyridin, F. 162-164°.

160. 4-(1-Benzothienyl-2-methylamino)-3-methyl-3H-imidazo(4,5-c)pyridin.

161. 4-(Naphthyl-1-methylamino)-3-methyl-3H-imidazo-(4,5-c)pyridin.

162. 4-(Naphthyl-2-methylamino)-3-methyl-3H-imidazo-(4,5-c)pyridin.

163. 4-m-Fluorbenzylamino-3-methyl-3H-imidazo(4,5-c)-pyridin.

164. 4-p-Fluorbenzylamino-3-methyl-3H-imidazo(4,5-c)-pyridin.

165. 4-o-Chlorbenzylamino-3-methyl-3H-imidazo(4,5-c)-pyridin, F. 179-180°.

166. 4-m-Chlorbenzylamino-3-methyl-3H-imidazo(4,5-c)-pyridin.

167. 4-p-Chlorbenzylamino-3-methyl-3H-imidazo(4,5-c)-pyridin.

168. 3-Methyl-4-o-nitrobenzylamino-3H-imidazo(4,5-c)-pyridin.

169. 3-Methyl-4-m-nitrobenzylamino-3H-imidazo(4,5-c)-pyridin.

170. 3-Methyl-4-p-nitrobenzylamino-3H-imidazo(4,5-c)-pyridin.

171. 3-Methyl-4-o-trifluormethylbenzylamino-3H-imidazo-(4,5-c)pyridin.

172. 3-Methyl-4-m-trifluormethylbenzylamino-3H-imidazo-(4,5-c)pyridin.

173. 3-Methyl-4-p-trifluormethylbenzylamino-3H-imidazo-(4,5-c)pyridin.

174. 4-(2,3-Dichlorbenzylamino)-3-methyl-3H-imidazo-(4,5-c)pyridin.

175. 4-(2,4-Dichlorbenzylamino)-3-methyl-3H-imidazo-(4,5-c)pyridin, F. 178-179°.

176. 4-(2,5-Dichlorbenzylamino)-3-methyl-3H-imidazo-(4,5-c)pyridin.

177. 4-(2,6-Dichlorbenzylamino)-3-methyl-3H-imidazo-(4,5-c)pyridin, F. 181-185°.
178. 4-(3,4-Dichlorbenzylamino)-3-methyl-3H-imidazo-(4,5-c)pyridin.
179. 4-(3,5-Dichlorbenzylamino)-3-methyl-3H-imidazo-(4,5-c)pyridin.
180. 4-(2,4-Dinitrobenzylamino)-3-methyl-3H-imidazo-(4,5-c)pyridin.
181. 4-(3,4-Dinitrobenzylamino)-3-methyl-3H-imidazo-(4,5-c)pyridin.
182. 4-(3,5-Dinitrobenzylamino)-3-methyl-3H-imidazo-(4,5-c)pyridin.
183. 4-(2-Chlor-6-fluorbenzylamino)-3-methyl-3H-imidazo-(4,5-c)pyridin.
184. 4-(2-Chlor-4-nitrobenzylamino)-3-methyl-3H-imidazo-(4,5-c)pyridin.
185. 4-(2-Chlor-5-nitrobenzylamino)-3-methyl-3H-imidazo-(4,5-c)pyridin.
186. 4-(4-Chlor-2-nitrobenzylamino)-3-methyl-3H-imidazo-(4,5-c)pyridin.
187. 4-(4-Chlor-3-nitrobenzylamino)-3-methyl-3H-imidazo-(4,5-c)pyridin.
188. 4-(5-Chlor-2-nitrobenzylamino)-3-methyl-3H-imidazo-(4,5-c)pyridin.

**Beispiel 189**

Ein Gemisch von 17,9 g 1-Methyl-4-methylthio-1H-imidazo-(4,5-c)pyridin (F.170-175°) und 28 g o-Chlorbenzylamin wird 20 Stunden auf 170-180° erhitzt. Nach dem Abkühlen und üblicher Aufarbeitung erhält man 4-o-Chlorbenzyl-amino-1-methyl-1H-imidazo(4,5-c)-pyridin, F. 173- 176°

**Beispiel 190**

Ein Gemisch von 2,81 g 4-(1-Benzothienyl-3-methoxy)-1H-imidazo(4,5-c)pyridin (erhältlich aus 1-Henzothienyl-3-methanol und 4-Chlor-1H-imidazo(4,5-c)pyridin), 1,7 g Methyljodid, 1,7 g Kaliumcarbonat und 50 ml Aceton wird zwei Tage bei 20° gerührt. Man saugt ab, dampft das Filtrat ein, arbeitet wie üblich auf und erhält 4-(1-Benzothienyl-3-methoxy)-1-methyl-1H-imidazo-(4,5-C) pyridin, F.200 - 204°.

**Beispiele 191 bis 377**

Analog Beispiel 190 erhält man aus den entsprechenden in 1- bzw. 3-Stellung unsubstituierten Imidazopyridinen durch Alkylierung die in den Beispielen 2 bis 188 angegebenen Verbindungen.

**Beispiel 378**

Ein Gemisch von 2,85 g 3-Amino-2-(1-benzothienyl-3-methoxy)-4-methylamino-pyridin (erhältlich aus 3-Amino-2-Chlor-4-methylamino-pyridin und 1-Benzothienyl-3-methanol) und 0,27 g s-Triazin wird eine Minute auf 210° gehalten und abgekühlt. Nach Umkristallisation aus Ethylacetat/Tetrahydrofuran erhält man 4-(1-Benzo-thienyl-3-methoxy)-1-methyl-1H-imidazo(4,5-c)pyridin, F. 200-204°

**Beispiele 379 bis 565**

Analog Beispiel 378 erhält man aus den entsprechenden Verbindungen der allgemeinen Formel VI mit s-Triazin die in den Beispielen 2 bis 188 angegebenen Verbindungen.

**Beispiel 566**

Ein Gemisch von 28,5 g 3-Amino-2-(1-benzothienyl-3-methoxy)-4-methylamino-pvridin, 100 ml Orthoameisensäuretriethylester und 40 ml Acetanhydrid wird 30 Minuten auf 100° erhitzt. Nach dem Eindampfen und üblicher Aufarbeitung erhält man 4-(1-Benzothienyl-3-mathoxy)-1-methyl-1H-imidazo(4,5-c)ayridin, F. 200-204°.

**Beispiele 567 bis 753**

Analog Beispiel 588 erhält man aus den entsprechenden Verbindungen der allgemeinen Formel VI mit Orthoameisensäuretriethylester die in den Beispielen 2 bis 188 angegebenen Verbindungen.

**Beispiele 754 bis 762**

Analog Beispiel 1 erhält man:
754. 4-(1-Benzothienyl-3-methoxy)-1-ethyl-1H-imidazo-(4,5-c)pyridin, F. 101-104°.
755. 4-(1-Benzothienyl-3-methoxy)-1-propyl-1H-imidazo-(4,5-c)pyridin, Hemifumarat, F. 106-110°.
756. 4-o-Chlorbenzyloxy-1-ethyl-1H-imidazo(4,5-c)pyridin, F. 97-99°.
757. 4-(2,4-Dichlorbenzyloxy)-1-ethyl-1H-imidazo(4,5-c)-pyridin, F. 112-114°.
758. 4-(1-Benzothienyl-3-methoxy)-3-ethyl-3H-imidazo-(4,5-c)pyridin, F. 116-118°.
759. 4-(1-Benzothienyl-3-methoxy)-3-propyl-3H-imidazo-(4,5-c)pyridin, F. 95-97°.
760. 4-(1-Benzothienyl-3-methoxy)-3-butyl-3H-imi-dazo(4,5-c)pyridin, F. 88-89°.
761. 4-(2,4-Dichlorbenzyloxy)-3-ethyl-3H-imidazo-(4,5-c)pyridin, F. 105-107°.
762. 4-(2,4-Dichlorbenzyloxy)-3-propyl-3H-imidazo-(4,5-c)pyridin, F. 107-109°.

**Beispiele 783 bis 774**

Analog Beispiel 83 erhält man:

763. 4-(1-Benzothienyl-3-methylthio)-1-ethyl-1H-imidazo(4,5-c)pyridin, F. 127-129°.

764. 4-(1-Benzothienyl-3-methylthio)-1-propyl-1H-imidazo(4,5-c)pyridin, F. 118-121°.

765. 4-o-Chlorbenzylthio-1-ethyl-1H-imidazo(4,5-c)-pyridin, F. 103-106°.

766. 4-o-Chlorbenzylthio-1-propyl-1H-imidazo(4,5-c)-pyridin, F. 72-76°.

767. 4-(2,4-Dichlorbenzylthio)-1-ethyl-1H-imidazo(4,5-c)-pyridin, F. 152-154°.

768. 4-(2,4-Dichlorbenzylthio)-1-propyl-1H-imidazo-(4,5-c)pyridin, F. 126-128°.

769. 4-(1-Benzothienyl-3-methylthio)-3-ethyl-3H-imidazo(4,5-c)pyridin,.F. 87-89°.

770. 4-(1-Benzothienyl-3-methylthio)-3-propyl-3H-imidazo(4,5-c)pyridin, F. 85-87°.

771. 4-(1-Benzothienyl-3-methylthio)-3-butyl-3H-imidazo(4,5-c)pyridin, Hemifumarat, F. 110-112°.

772. 4-(1-Benzothienyl-3-methylthio)-3-cyclopropyl-methyl-3H-imidazo(4,5-c)pyridin, F. 135-136°.

773. 4-o-Chlorbenzylthio-3-ethyl-3H-imidazo(4,5-c)-pyridin, Hemifumarat, F. 126-130°.

774. 4-o-Chlorbenzylthio-3-propyl-3H-imidazo(4,5-c)-pyıidin, Dihydrochlorid, F. 125-132°.

Die nachstehenden Beispiele betreffen pharmazeutische Zubereitungen, die Verbindungen der Formel I oder ihre Säureadditionssalze enthalten:

**Beispiel A: Tabletten**

Ein Gemisch von 1 kg 4-(1-Benzothienyl-3-methoxy)-3-methyl-3H-imidazo(4,5-c)pyridin ("B"), 4 kg Lactose, 1,2 kg Kartoffelstärke, 0,2 kg Talk und 0,1 kg Magnesiumstearat wird in üblicher Weise zu Tabletten gepreßt, derart, daß jede Tablette 100 mg Wirkstoff enthält.

**Beispiel B: Dragees**

Analog Beispiel A werden Tabletten gepreßt, die anschliesend in üblicher Weise mit einem Überzug aus Saccharose, Kartoffelstärke, Talk, Tragant und Farbstoff überzogen werden.

**Beispiel C: Kapseln**

10 kg "B" werden in üblicher Weise in Hartgelatinekapseln gefüllt, so daß jede Kapsel 50 mg Wirkstoff enthält.

**Beispiel D: Ampullen**

Eine Lösung von 1 kg "B"-Hydrochlorid in 100 l zweifach destilliertem Wasser wird steril filtriert, in Ampullen abgefüllt, unter sterilen Bedingungen lyophilisiert und steril verschlossen. Jede Ampulle enthält 50 mg Wirkstoff.

Analog sind Tabletten, Dragees, Kapseln und Ampullen erhältlich, die einen oder mehrere der übrigen Wirkstoffe der Formel I und/oder ihre physiologisch ungedenklichen Säureadditionssalze enthalten.

**Patentansprüche:**

1. Imidazo(4,5-c)pyridine der allgemeinen Formel I

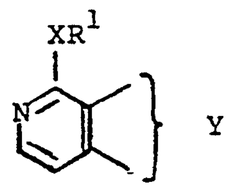 I

worin

X O, S oder NH,

Y $-NR^2-CH=N-$ oder $-N=CH-NR^2$

$R^1$ 1-Benzothienyl-methyl, Naphthylmethyloder ein- oder zweifach durch F, Cl, $NO_2$ und/oder $CF_3$ substituiertes Benzyl und

$R^2$ Alkyl mit 1- 4 C-Atomen oder Cyclopropylmethyl

bedeuten,

sowie deren physiologisch unbedenkliche Säureadditionssalze.

2. 4-(1; Benzothienyl-3-methoxyl-3-methyl-3H-imidazo-(4,5-c)pyridin.

3. 4-c-Chlorbenzyloxy-1-methyl-1H-imidezo(4,5-c)pyridin.

4. Verfahren zur Herstellung von Imidazo(4,5-c)-pyridinen der allgemeinen Formel I gemäß Anspruch 1

sowie von ihren physiologisch unbedenklichen Säureadditionssalzen, dadurch gekennzeichnet, daß man eine Verbindung der allgemeinen Formel II

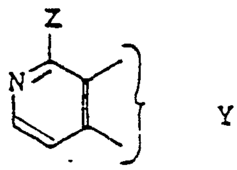

II

worin

Z F, Cl, Br, J, OH, SH, $SR^3$, $SOR^3$, $SO_2R^3$ oder $NH_2$ und

$R^3$ Alkyl mit 1 - 4 C-Atomen, Phenyl oder Benzyl bedeuten und

Y die angegebene Bedeutung hat oder eines ihrer reaktionsfähigen Derivate mit einer Verbindung der allgemeinen Formel III

$HX-R^1$ III

worin

X und $R^1$ die angegebene Bedeutung haben oder einem ihrer reaktionsfähigen Derivate umsetzt

oder daß man eine Verbindung der allgemeinen Formel IV

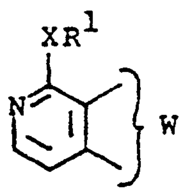

IV

worin

W -NH-CH=N- oder -N=CH-NH- bedeutet

mit einer Verbindung der allgemeinen Formel V

$Z-R^2$ V

worin

$R^2$ und Z die angegebene Bedeutung haben oder einem ihrer reaktionsfähigen Derivate umsetzt

oder daß man eine Verbindung der allgemeinen Formel VI

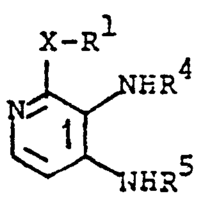

VI

worin

der eine der Reste $R^4$ bzw. $R^5$ H, der andere dieser Reste $R^2$ bedeutet und

X und $R^1$ die angegebene Bedeutung haben mit Ameisensäure oder einem ihrer reaktionsfähigen Derivate umsetzt

und/oder daß man gegebenenfalls eine Verbindung der allgemeinen Formel I durch Behandeln mit einer Säure in eines ihrer physiologisch unbedenklichen Säureadditionssalze umwandelt.

5. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

6. Pharmazeutische Zubereitung, gekennzeichnet durch einen Gehalt an mindestens einer Verbindung der Formel I und/oder einem ihrer physiologisch unbedenklichen Salze.

7. Verbindungen der allgemeinen Formel I nach patentanspruch 1 zur Bekämpfung von Krankheiten.

8. Verwendung von Verbindungen der allgemeinen Formel I und/oder ihrer physiologisch unbedenklichen Salze zu Herstellung von Arzneimitteln.

**Claims**

1. Imidazo(4,5-c)pyridines of the general formula I

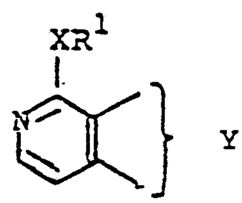

· I

wherein

X is O, S or NH,

Y is $-NR^2-CH=N-$ or $-N=CH-NR^2-$,

$R^1$ is l-benzothienylmethyl, naphthylmethyl, or benzyl which is mono-or di-substituted by F, Cl, $NO_2$ and/or $CF_3$ and

$R_2$ is alkyl with 1 - 4 C atoms or cyclopropylmethyl, and physiologically acceptable acid addition salts thereof.

2. 4-(1-Benzothienyl-3-methoxy)-3-methyl-3H-imidazo-(4,5-c)pyridine.

3. 4-o-Chlorobenzyloxy-1-methyl-1H-imidazo(4,5-c)-pyridine.

4. Process for the preparation of imidazo(4,5-c)-pyridines of the general formula I accordinq to Claim 1 and of their physiologically acceptable acid addition salts, characterised in that a compound of the general formula II

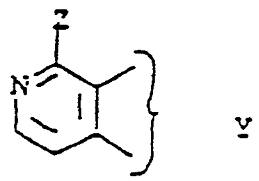

II

wherein

Z is F, Cl, Br, I, OH, SH, $SR^3$, $SOR^3$, $SO_2R^3$ or $NH_2$,

$R^3$ is alkyl with 1 - 4 C atoms, phenyl or benzyl and

Y has the meaning given,

or one of its reactive derivatives, is reacted with a compound of the general formula III

$HX-R^1$ III

wherein

X and $R^1$ have the meanings given, or one of its reactive derivatives,

or in that a compound of the general formula IV

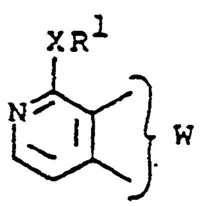

IV

wherein

W is -NH-CH=N- or -N=CH-NH-,

is reacted with a compound of the general formula V

$Z-R^2$ V

wherein

$R^2$ and Z have the meanings given, or one of its reactive derivatives,

or in that a compound of the general formula VI

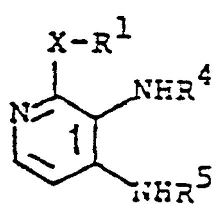

VI

wherein

one of the radicals $R^4$ or $R^5$ is H and the other of these radicals is $R^2$, and

X and $R^1$ have the meanings given,

is reacted with formic acid or one of its reactive derivatives, and/or in that, if appropriate, a compound of the general formula I is converted into one of its physiologically acceptable acid addition salts by treatment with an acid.

5. Process for the preparation of pharmaceutical formulations, characterised in that a compound of the formula I and/or one of its physiologically acceptable salts is brought into a suitable dosage form, together with at least one solid, liquid or semi-liquid excipient or auxiliary and, if appropriate, in combination with one or more other active compound(s).

6. Pharmaceutical formulation, characterised in that it contains at least one compound of the formula I and/or one of its physiologically acceptable salts.

7. Compounds of the general formula I according to Patent Claim 1, for combating illnesses.

8. Use of compounds of the general formula I and/or their physiologically acceptable salts for the manufacture of medicaments.

**Revendications**

1. Imidazo(4,5-c)pyridines de formule générale I:

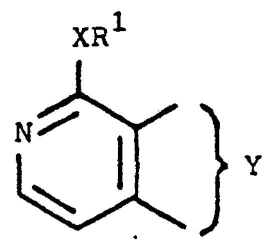

I

dans laquelle

X représente O, S ou NH,

Y représente $-NR^2-CH=N-$ ou $-N=CH-NR^2-$,

$R^1$ représente un groupe 1-benzothiényl-méthyle, un groupe naphtylméthyle ou un groupe benzyle substitué une ou deux fois par F, Cl, $NO_2$ et/ou $CF_3$, et

$R^2$ représente un groupe alkyle contenant 1 à 4 atomes de carbone ou un groupe cyclopropylméthyle, ainsi que leurs sels d'addition d'acide physiologiquement acceptables.

2. La 4-(1-benzothiényl-3-méthoxy)-3-méthyl-3H-imidazo(4,5-c)pyridine.

3. La 4-o-chlorobenzyloxy-1-méthyl-1H-imidazo(4,5-c)pyridine.

4. Procédé de préparation d'imidazo(4,5-c)-pyridines de formule générale I selon la revendication 1, ainsi que de leurs sels d'addition d'acide physiologiquement acceptables, caractérisé en ce qu'on fait réagir un composé de formule générale II:

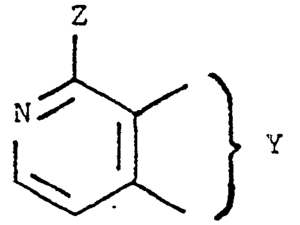

II

dans laquelle

Z représente F, Cl, Br, I, OH, SH, $SR^3$, $SOR^3$, $SO_2R^3$ ou $NH_2$, et

$R^3$ représente un groupe alkyle contenant 1 à 4 atomes de carbone, un groupe phényle ou un groupe benzyle, et

Y a la signification indiquée,ou un de ses dérivés réactifs, avec un composé de formule générale III:

$HX-R^1$ III

dans laquelle

X et $R^1$ ont les significations indiquées, ou de ses dérivés réactifs,

ou on fait réagir un composé de formule générale IV:

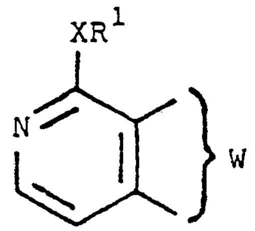

IV

dans laquelle

W représente -NH-CH=N- ou -N=CH-NH-, avec un composé de formule générale V:

$Z-R^2$ V

dans laquelle

$R^2$ et Z ont les significations indiquées, ou un de ses dérivés réactifs,

ou on fait réagir un composé de formule générale VI:

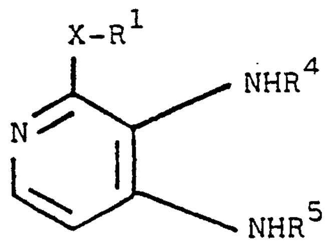

VI

dans laquelle

un des radicaux $R^4$ et $R^5$ représente H, l'autre radical représentant $R^2$, tandis que X et $R^1$ ont les significations indiquées, avec l'acide formique ou un de ses dérivés réactifs, et/ou on transforme éventuellement un composé de formule générale I en un de ses sels d'addition d'acide physiologiquement acceptables par traitement avec un acide.

5. Procédé en vue d'obtenir des préparations pharmaceutiques, caractérisé en ce qu'on améne, sous une forme de dosage appropriée, un composé de formule I et/ou un de ses sels physiologiquement acceptables conjointement avec au moins une substa ce auxiliaire ou une substance support solide, liquide ou semi-liquide et éventuellement en combinaison avec une ou plusieurs autres substa ces actives.

6. Préparation pharmaceutique, caractérisée en ce qu'elle contient au moins un composé de formule I et/ou un de ses sels physiologiquement acceptables.

7. Composés de formule générale I selon la revendication I en vue de combattre les maladies.

8. Utilisation de composés de formule générale I et/ou de leurs sels physiologiquement acceptables en vue de préparer des médicaments.